# FASCICULE DE BREVET EUROPEEN

(11) **EP 1 220 651 B1**
(45) Date de publication et mention de la délivrance du brevet: **31.03.2004**
(21) Numéro de dépôt: 00967996.0
(22) Date de dépôt: 10.10.2000
(51) Int. Cl.: A61F 2/24

(54) **DISPOSITIF D'ANNULOPLASTIE UTILISABLE PAR VOIE MINI-INVASIVE**
ANNULOPLASTIEVORRICHTUNG ZUR MINIMAL-INVASIVEN VERWENDUNG
ANNULOPLASTY DEVICE FOR USE IN MINIMALLY INVASIVE PROCEDURE

(30) Priorité: 12.10.1999 FR 9912976
(43) Date de publication de la demande: 10.07.2002
(73) Titulaire: Seguin, Jacques, 75008 Paris (FR)
(72) Inventeur: Seguin, Jacques, 75008 Paris (FR)
(86) Numéro de dépôt international: PCT/FR2000/002814
(87) Numéro de publication internationale: WO 2001/026586

(56) Documents cités:
- EP-A- 0 667 133
- WO-A-93/15690
- WO-A-98/57599
- US-A- 5 716 397
- US-A- 5 824 066

## Description

La présente invention concerne un dispositif d'annuloplastie utilisable par voie mini-invasive notamment pour la reconstruction de valves cardiaques.

Dans une valve cardiaque normale, les valvules se recouvrent au centre de l'anneau fibro-musculaire qui entoure la valve, et assurent ainsi l'étanchéité de la valve contre une régurgitation du sang.

Différentes affections peuvent conduire à des déformations ou des dilatations de cet anneau fibro-musculaire, qui génèrent un défaut d'étanchéité des valvules.

Il est alors nécessaire de "reconstruire" la valve par la mise en place d'un implant venant redonner à l'anneau fibro-musculaire une forme et/ou une section correctes, cette opération étant couramment dénommée "annuloplastie".

Un tel implant présente de manière bien connue en soi une forme annulaire et est suturé à l'anneau fibro-musculaire. Il peut avoir une structure rigide, semi-rigide ou souple.

L'introduction et la mise en place de ce genre d'implants exigent une large voie d'abord, ce qui rend ces opérations longues, complexes et délicates.

La chirurgie mini-invasive permet certes de réduire la lourdeur du geste opératoire en diminuant la voie d'abord jusqu'à la limiter à une ou plusieurs ouvertures ayant 1 à 3 cm de diamètre. Cette chirurgie n'est toutefois pas utilisable actuellement pour une annuloplastie, un anneau de reconstruction valvulaire ne passant pas par de telles ouvertures, en particulier s'il est rigide ou semi-rigide. De plus, un tel anneau et l'instrument permettant l'introduction de cet anneau seraient de toute façon très difficiles à manipuler, et l'engagement de fils de suture dans l'anneau, une fois ce dernier placé dans le coeur, serait problématique.

La présente invention vise à remédier à ces inconvénients fondamentaux.

Son objectif principal est de fournir un dispositif qui permette une annuloplastie par voie très peu invasive, c'est-à-dire par une ou plusieurs voies d'abord formées par des passages ayant de l'ordre de un à deux centimètres de diamètre.

Un autre objectif de l'invention est de fournir un dispositif qui permette une réalisation facilitée des sutures de fixation de l'anneau au site à traiter.

Le dispositif concerné comprend, de manière connue en soi, un implant conformé pour pouvoir être suturé à des tissus afin de réaliser l'annuloplastie.

Selon l'invention :
- l'implant a une structure allongée et déformable, telle qu'il peut prendre d'une part une forme allongée pour son introduction dans le corps du patient par un passage de diamètre réduit, de l'ordre d'un à deux centimètres, et d'autre part une forme courbe adaptée à la réalisation de l'annuloplastie, et
- le dispositif comprend un instrument tubulaire propre à recevoir au moins partiellement ledit implant en lui, qui présente une rigidité telle qu'il permet l'introduction de l'implant dans le corps du patient par ledit passage ; cet instrument comprend une ouverture au niveau de sa partie distale, permettant l'accès à l'implant, et comprend des moyens de calage en rotation de l'implant par rapport à lui, des moyens de maintien de l'implant par rapport à lui, et des moyens de repérage de son orientation angulaire à l'intérieur du corps du patient.

Les moyens de calage et de repérage permettent, par l'orientation adéquate de l'instrument par rapport auxdits tissus, de positionner l'implant de manière adéquate par rapport à ces tissus, en vue de la réalisation de l'annuloplastie grâce à ladite forme courbe de l'implant ; ces moyens de calage ainsi que les moyens de maintien permettent de maintenir une portion de l'implant en position adéquate par rapport aux tissus lors de la mise en place du fil permettant de suturer cette portion à ces tissus, puis de libérer cette portion de l'implant une fois qu'elle a été suturée aux tissus.

En pratique, un trocart contenant un tube est introduit jusqu'à ce que la partie distale de ce trocart et de ce tube se trouvent à proximité du site à traiter ; le trocart est retiré et le tube permet alors l'introduction de l'instrument, à l'intérieur duquel est maintenu l'implant ; la partie distale de l'implant, accessible au niveau de la partie distale de l'instrument, est fixée aux tissus par un premier point de suture ; une portion supplémentaire de l'implant est alors délivrée par l'instrument puis cette portion est fixée auxdits tissus par un deuxième point de suture ; une nouvelle portion de l'implant est ensuite libérée puis suturée, et ainsi de suite jusqu'à ce que l'ensemble de l'implant soit fixé aux tissus.

L'implant pourrait avoir une structure élastique et présenter la forme courbe précitée à l'état non déformé de cette structure ; cet implant serait alors déformé élastiquement lors de son engagement dans l'instrument puis retrouverait sa forme courbe au fur et à mesure de sa délivrance par l'instrument ; les moyens de calage et de repérage que comprend l'instrument permettraient alors de maintenir l'implant au cours de la suture de telle sorte que la partie concave de l'implant soit située radialement vers l'intérieur de l'annuloplastie à réaliser.

L'implant pourrait également être réalisé en alliage à mémoire de forme tel qu'un alliage Nickel-titane connu sous la dénomination "NITINOL".

De préférence toutefois, l'implant comprend un corps ayant une structure souple non élastique, et au moins un cordon relié audit corps, au voisinage de l'une des extrémités de ce dernier ; chaque cordon s'étend sur un côté longitudinal de ce corps jusqu'à un emplacement éloigné de ladite extrémité, est monté coulissant par rapport à ce corps et par rapport audit emplacement, et présente une longueur telle qu'une traction peut être opérée sur lui après la suture dudit corps auxdits tissus ; l'ensemble est conformé de telle sorte qu'une traction peut être opérée sur chaque cordon afin de réduire la longueur dudit corps, en fronçant ladite structure, de manière à réduire la circonférence de l'implant et, ce faisant, à réaliser l'annuloplastie.

Lesdits moyens de repérage permettent dans ce cas le maintien de l'implant dans une position dans laquelle chaque cordon est positionné sur le côté radialement interne du corps au moment de la suture de l'implant.

Une fois la suture de l'implant réalisée, une traction est opérée sur le ou les cordons de manière à froncer et donc à raidir ledit corps puis ce ou ces cordons sont suturés afin d'être maintenus en traction.

Selon une forme de réalisation possible de l'invention dans ce cas, ledit corps est constitué par une tresse de matière textile, chaque cordon passant simplement à l'intérieur de cette tresse.

L'implant peut comprendre un seul cordon, relié à une extrémité du corps et débouchant au voisinage de l'autre extrémité de celui-ci ; de préférence toutefois, l'implant comprend deux cordons, dont un est relié à une extrémité du corps et l'autre à l'autre extrémité de ce corps, et les deux cordons s'étendent chacun sur sensiblement la moitié du corps, jusqu'à des emplacements situés à proximité l'un de l'autre sensiblement dans la zone médiane de ce corps.

Selon une forme de réalisation possible de l'invention, l'instrument présente une encoche latérale au voisinage de son extrémité distale, qui communique par une fente avec l'ouverture distale de cet instrument, la profondeur de cette encoche étant telle qu'elle permet de découvrir le côté longitudinal du corps destiné, après suture, à être situé radialement vers l'extérieur de l'annuloplastie à réaliser, mais telle qu'elle recouvre le côté longitudinal du corps destiné à être situé sur le côté radialement intérieur de cette annuloplastie, c'est-à-dire sur le côté sur lequel se trouvent le ou les cordons.

Cette encoche permet par conséquent de ne découvrir que la partie du corps devant être suturée aux tissus et élimine tout risque que le ou les cordons soient pris par l'aiguille de suture.

De préférence, des moyens de maintien tels que précités sont situés de part et d'autre de cette encoche, pour parfaitement raidir l'implant au niveau de l'encoche et faciliter ainsi le passage du fil de suture.

L'instrument peut alors comprendre deux pièces tubulaires dont une première est engagée dans la deuxième ; ladite première pièce tubulaire comporte des dents au niveau de son extrémité distale, constituant les moyens de calage et les moyens de maintien précités ; ces dents sont mobiles radialement entre une position normale, radialement externe, dans laquelle elles autorisent le coulissement de l'implant et une position radialement interne, dans laquelle elles serrent l'implant entre elles et empêchent ce coulissement, et ces dents sont conformées de telle sorte que leurs faces radialement extérieures fassent saillie, dans ladite position normale, au-delà de la face extérieure de ladite première pièce tubulaire ; la deuxième pièce tubulaire peut coulisser axialement par rapport à la première pièce tubulaire, entre une position effacée, dans laquelle elle ne porte pas contre lesdites faces radialement extérieures des dents, et une position active, dans laquelle elle porte contre ces faces radialement extérieures et déplace les dents dans leur position radialement interne.

Selon une deuxième forme de réalisation possible de l'invention, le corps de l'implant présente, vu en coupe transversale, une partie tubulaire et une partie plane s'étendant radialement par rapport à cette partie tubulaire ; l'instrument comprend une pièce tubulaire qui présente une fente latérale aménagée dans sa partie distale, et comprend une tige pouvant être engagée dans cette pièce tubulaire ; ladite partie tubulaire du corps de l'implant est engagée à l'intérieur de cette pièce tubulaire de l'instrument et reçoit ladite tige en elle tandis que ladite partie plane traverse ladite fente et débouche sur l'extérieur de ladite pièce tubulaire de l'instrument.

Cette fente et cette tige constituent lesdits moyens de calage et de maintien de l'implant dans l'instrument. La libération de l'implant s'effectue simplement en retirant la tige au fur et à mesure de la fixation des portions de l'implant aux tissus, afin de permettre le passage de la partie tubulaire du corps de l'implant au travers de ladite fente.

L'instrument peut comprendre deux pièces tubulaires, à savoir la pièce tubulaire précitée et une pièce tubulaire externe, dans laquelle sont engagés la pièce tubulaire précitée et l'implant placé dans celle-ci.

Avantageusement, la portion de ladite partie plane du corps de l'implant s'étendant au-delà de l'instrument comprend les moyens de fixation de l'implant aux tissus, qui sont pré-positionnés sur elle.

Ces moyens sont en particulier constitués par des fils de suture.

Ce pré-positionnement desdits moyens de fixation, rendu possible par l'existence de ladite partie plane, facilite très notablement la réalisation de la fixation de l'implant aux tissus.

De préférence, dans ce cas, les moyens de fixation sont constitués par des fils de suture et l'implant présente, en regard de chaque fil de suture, au moins un bobinot monté sur lui, sur lequel le fil de suture est enroulé. L'aiguille de suture reliée à ce fil peut également être maintenue sur ce bobinot le temps qu'elle soit utilisée.

Lorsque les fils de suture traversent ladite partie plane, et forment par conséquent chacun deux brins qui s'étendent de part et d'autre de cette partie plane, l'instrument présente avantageusement un bobinot sur chacun de ses côtés situés de part et d'autre de cette partie plane, chacun des bobinots recevant l'un des deux brins du fil de suture.

Pour sa bonne compréhension, l'invention est à nouveau décrite ci-dessous en référence au dessin schématique annexé représentant, à titre d'exemples non limitatifs, deux formes de réalisation possibles du dispositif d'annuloplastie qu'elle concerne.
La figure 1 est une vue de côté d'un implant et d'un instrument tubulaire que comprend ce dispositif, selon une première forme de réalisation ;
la figure 2 est une vue en perspective de cet implant et de cet instrument, l'implant étant placé à l'intérieur de l'instrument ;
la figure 3 est une vue de côté de cet implant et de cet instrument, en coupe longitudinale ;
la figure 4 est une vue très simplifiée d'une valve cardiaque sur laquelle une annuloplastie doit être réalisée, ainsi que desdits implant et instrument ;
les figures 5 à 8 sont des vues agrandies, très simplifiées, de cette valve cardiaque et de ces implant et instrument, au cours de quatre étapes successives de mise en place d'un point de suture de l'implant à un tissu de la valve ;
la figure 9 est une vue similaire à la figure 4 après mise en place de l'implant et avant réalisation de l'annuloplastie de la valve ;
la figure 10 est une vue de côté, avec coupe partielle d'un implant et d'une partie d'un instrument que comprend ce dispositif, selon une deuxième forme de réalisation ;
la figure 11 en est une vue en coupe selon la ligne XI-XI de la figure 10, et
la figure 12 est une vue partielle, en perspective, d'un implant et d'une partie de l'instrument selon cette deuxième forme de réalisation, l'implant étant équipé de bobinots.

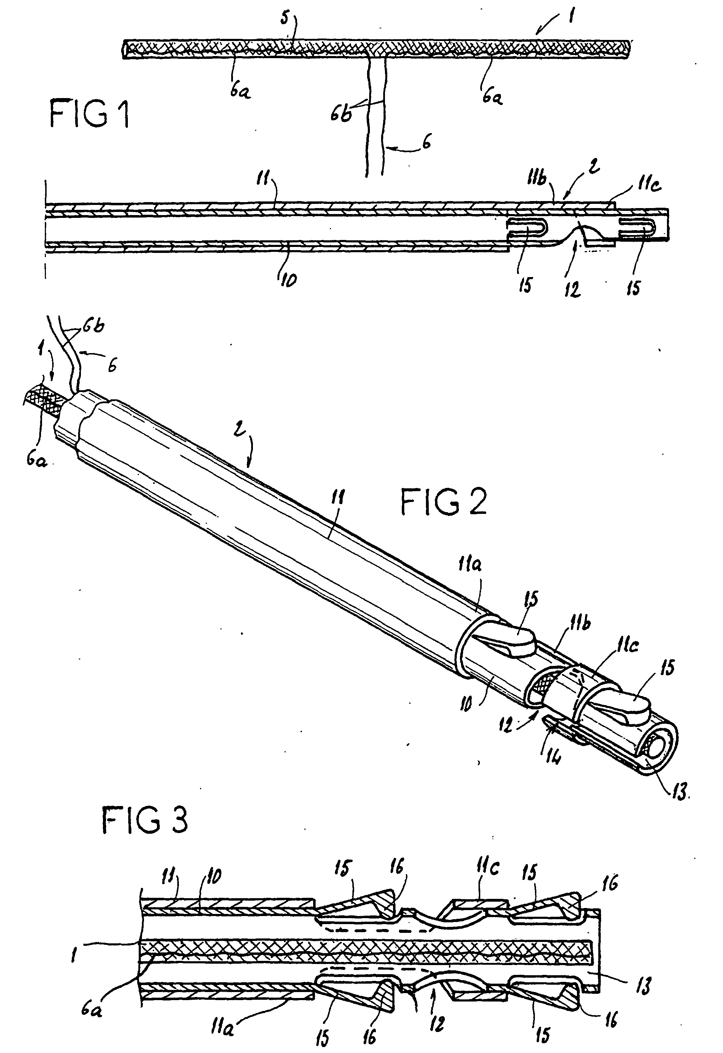

La figure 1 représente un implant 1 permettant de réaliser une annuloplastie sur une valve cardiaque et un instrument tubulaire 2 permettant la mise en place de cet implant 1.

L'implant 1 comprend un corps 5 constitué par une tresse tubulaire en matière textile, par exemple en polyester, et par deux cordons 6.

Les extrémités du corps 5 sont ligaturées, de manière à maintenir ensemble les brins de la tresse.

L'une des extrémités d'un cordon 6 est reliée, par ladite ligature, à une des extrémités de la tresse tandis que l'extrémité de l'autre cordon 6 est reliée, par l'autre ligature, à l'autre des extrémités de cette même tresse. Ces deux cordons 6 présentent chacun une partie 6a qui s'étend à l'intérieur du corps 5, le long d'un côté longitudinal de celui-ci, jusqu'au voisinage de la portion médiane de ce corps 5, et une portion 6b, qui s'étend en dehors de ce corps 5, chaque cordon 6 traversant ce corps 5 et sortant de celui-ci au niveau de ladite portion médiane. Les deux cordons 6 peuvent coulisser dans la tresse et au travers des ouvertures par lesquelles ils traversent cette tresse.

La longueur de ces cordons 6 est telle que chacun des cordons 6 débouche à l'extérieur du corps du patient lorsque l'implant 1 est fixé à la valve, ainsi que cela sera décrit plus loin.

L'instrument 2 comprend deux pièces tubulaires 10, 11 engagées l'une dans l'autre.

La pièce 10 radialement intérieure présente une encoche latérale 12 au voisinage de son extrémité distale, qui communique avec l'ouverture distale 13 de cette pièce par une fente 14. La largeur de cette fente 14 est telle qu'un point de suture permettant de fixer le corps 5 à la valve cardiaque peut passer au travers d'elle. La profondeur de l'encoche 12 est telle que cette encoche permet de découvrir le côté longitudinal du corps 5 opposé à celui sur lequel se trouvent les cordons 6 lorsque ceux-ci sont tendus mais telle que l'encoche recouvre ce côté longitudinal sur lequel se trouvent lesdits cordons 6.

Cette pièce 10 comprend également deux paires de dents 15, chaque paire étant située longitudinalement sur un côté de l'encoche 12. La pièce 10 est réalisée en une matière synthétique moulable, présentant un certain degré de souplesse, et chaque dent 15 est réalisée dans le même matériau que cette pièce 10, par moulage, en étant individualisée par rapport à la pièce 10 au moyen d'une fente en forme de U. Les dents 15 de chaque paire de dents sont diamétralement opposées et chaque dent 15 présente une partie 16 d'appui contre l'implant 1, faisant saillie radialement vers l'intérieur de la pièce 10.

Chaque dent 15 occupe normalement la position montrée en traits pleins à la figure 3, dans laquelle elle est inclinée vers l'extérieur de la pièce 10 de telle sorte que sa partie d'appui 16 est située à distance du corps 5 de l'implant 1.

Cette même figure montre que la souplesse du matériau constituant la pièce 10 permet une flexion de ces dents 15 vers une position radialement intérieure, dans laquelle les parties 16 viennent serrer l'implant 1 entre elles. Ce serrage écrase le corps 5 et permet d'immobiliser ce dernier par rapport à l'instrument 2, tant en rotation qu'en coulissement par rapport à celui-ci. Ce serrage permet également de maintenir les cordons 6 légèrement en tension, pour qu'ils soient maintenus le long du côté longitudinal précité de ce corps 5.

La pièce 11 est engagée sur la pièce 10 et peut coulisser axialement par rapport à elle. Elle présente une partie tubulaire proximale 11a, un tenon intermédiaire 11b et une partie tubulaire distale 11c qui est fendue longitudinalement. Il apparaît aux figures 2 et 3 que les parties 11a et 11c sont propres, dans une première position axiale par rapport à la pièce 10, à recouvrir respectivement les dents 15 proximales et distales, de manière à déplacer ces dents 15 vers leur position radialement interne précitée, et, dans la position axiale représentée, à être positionnées en dehors de ces dents 15, de telle sorte que celles-ci viennent occuper leur position radialement extérieure normale.

Le tenon 11b présente une longueur telle que la partie 11c ne vient en aucune manière recouvrir l'encoche 12 dans ladite position.

En outre, la pièce 11 est calée en rotation par rapport à la pièce 10, par exemple par des tétons coulissant dans des rainures, de telle sorte que la fente de la partie 11c coïncide avec la fente 14.

Au niveau proximal, l'instrument 2 comprend une marque permettant de repérer le côté de cet instrument sur lequel se trouve l'encoche 12 et d'orienter angulairement cet instrument 12.

En pratique, l'implant 1 est mis en place dans l'instrument 2 de telle sorte que la partie distale de l'implant 1 se trouve au niveau de l'ouverture distale 13 de l'instrument et que les cordons 6 s'étendent le long du côté de la pièce 10 opposé à celui sur lequel débouche l'encoche 12. La pièce 11 est ensuite déplacée par rapport à la pièce 10, ce qui amène les dents 15 vers leur position radialement interne et immobilise ainsi l'implant 1 par rapport à l'instrument 2, tant axialement qu'en rotation, tout en maintenant le corps 5 tendu dans l'encoche 12.

Pour la mise en place de l'implant 1, une incision est pratiquée en un emplacement adéquat de la cage thoracique du patient, puis un trocart contenant un tube est engagé sous la peau du patient, ainsi qu'au travers de la graisse et des tissus, jusqu'aux abords de la valve cardiaque à traiter.

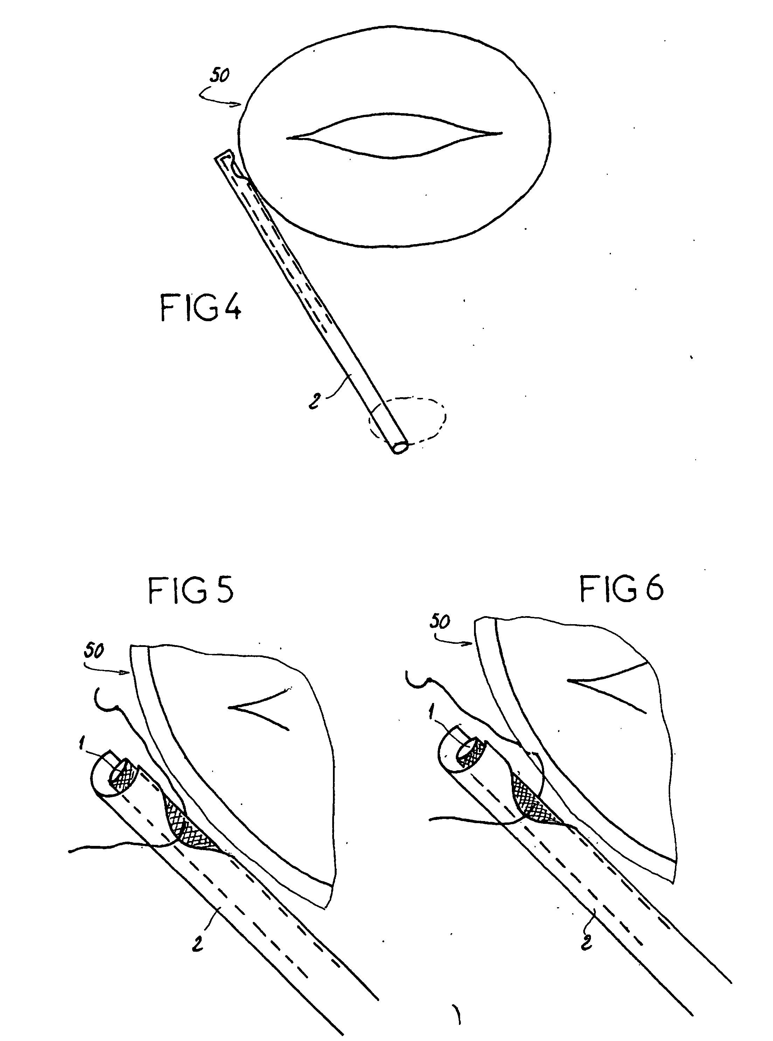

Le trocart est alors retiré et l'ensemble implant 1-instrument 2 est engagé dans le corps du patient au moyen dudit tube, puis convenablement orienté au moyen du repère proximal précité (figure 4).

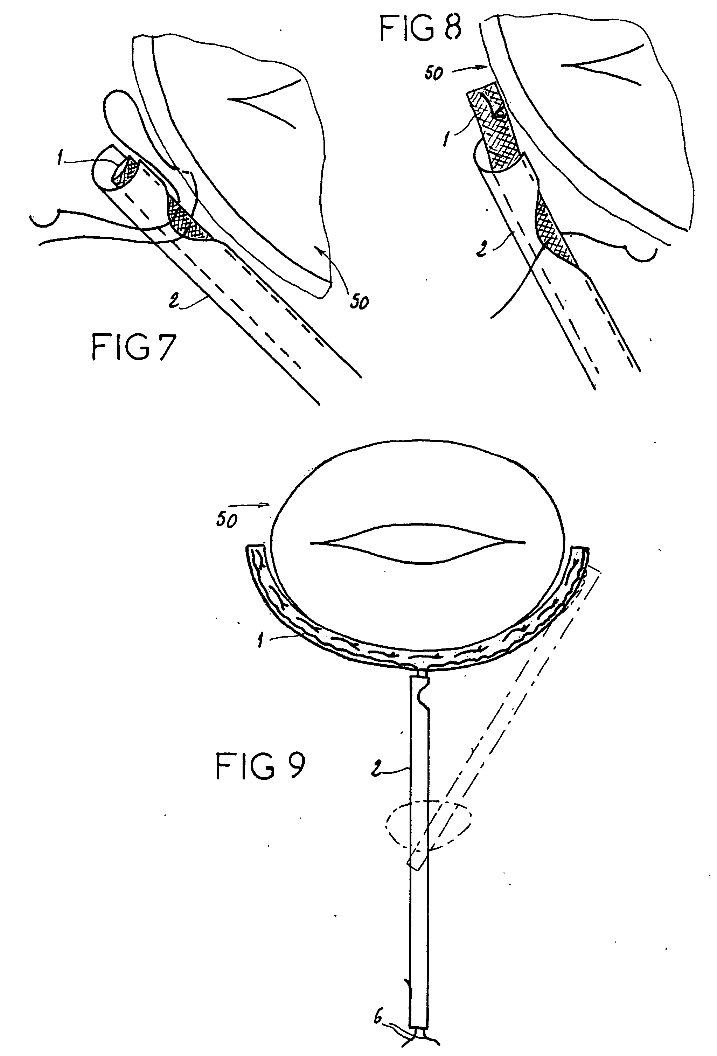

Ainsi que le montrent les figures 5 à 7, le fil de suture est passé successivement dans l'implant 1 au niveau de l'encoche 12 (figure 5) puis dans l'anneau fibro-musculaire qui entoure la valve 50 à traiter (figure 6) puis à nouveau dans l'implant 1 au niveau de l'encoche 12 (figure 7). La pièce 11 est alors reculée par rapport à la pièce 10, pour libérer le coulissement de l'implant 1 de manière à délivrer une portion supplémentaire de cet implant 1. Le fil de suture est alors noué afin de solidariser l'implant à l'anneau fibro-musculaire.

La pièce 11 est ensuite à nouveau avancée pour immobiliser l'implant 1 dans l'instrument 2 puis un fil de suture est à nouveau passé dans l'implant 1 au niveau de l'encoche 12 (figure 8) et dans l'anneau fibro-musculaire ; le coulissement de l'implant est alors à nouveau libéré pour le nouage de ce fil de suture et la délivrance d'une portion supplémentaire de l'implant 1.

Ces opérations sont répétées le nombre de fois nécessaire pour la fixation de l'ensemble de l'implant 1 autour de tout ou partie de la valve 50.

Une fois l'implant 1 fixé à l'anneau fibro-musculaire, une traction est opérée sur les cordons 6 afin de réduire la longueur du corps 5, en fronçant sa structure, pour réduire la circonférence de l'implant 1 et, ce faisant, à réaliser l'annuloplastie ; les cordons 6 sont alors noués l'un à l'autre et un instrument "pousse-noeud" est utilisé pour amener le noeud au niveau du corps 5, afin que les cordons 6 soient maintenus en traction.

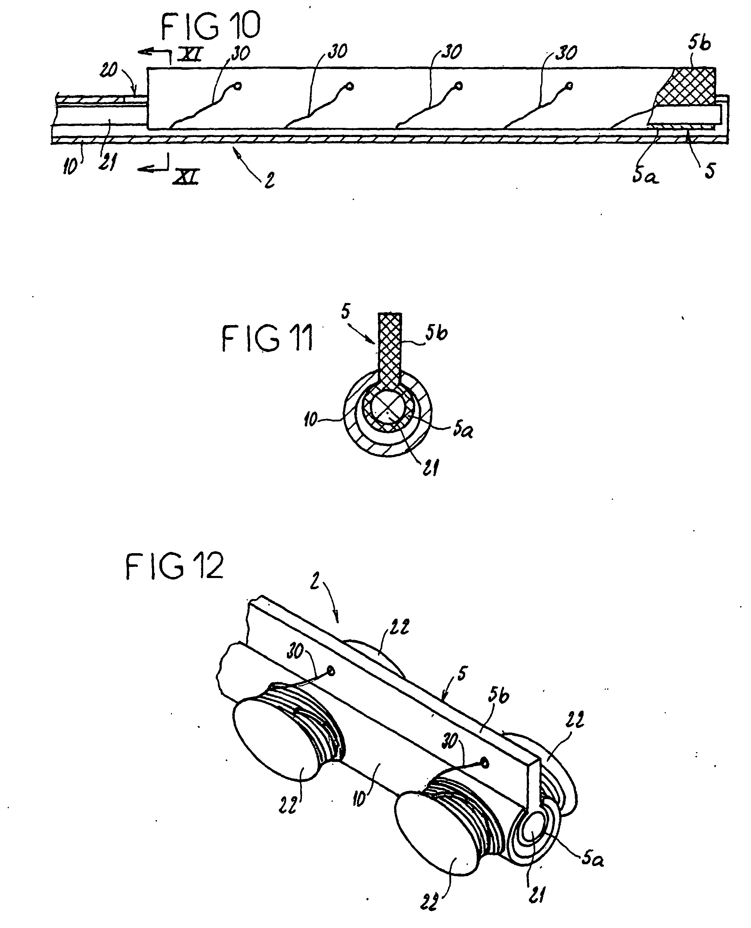

Les figures 10 à 12 représentent un implant dont le corps 5 est également constitué par une tresse en matériau textile et est équipé de deux cordons de rigidification tels que précités, non représentés.

Ainsi que le montrent ces figures, le corps 5 présente, vu en coupe transversale, une partie tubulaire 5a et une partie plane 5b s'étendant radialement par rapport à cette partie tubulaire 5a. La partie plane 5b est traversée par une pluralité de fils de suture 30, placés de proche en proche, sur les extrémités desquels sont serties des aiguilles de suture.

L'instrument 2 comprend une pièce tubulaire interne 10 qui présente une fente latérale 20 aménagée dans sa partie distale, et comprend une tige 21 pouvant être engagée dans la pièce 10, cette tige 21 ayant un diamètre supérieur à la largeur de la fente 20.

Il apparaît que la partie 5a de l'implant est engagée à l'intérieur de la pièce 10 et reçoit la tige 21 en elle tandis que la partie 5b traverse la fente 20 et débouche sur l'extérieur de la pièce 10.

L'instrument 2 comprend également une pièce tubulaire extérieure (non représentée), dans laquelle sont engagés la pièce tubulaire 10 et l'implant placé dans celle-ci.

La fente 20 et la tige 21 constituent des moyens de calage et de maintien de l'implant 1 dans l'instrument 2. La libération de l'implant 1 s'effectue simplement en retirant ladite pièce tubulaire extérieure puis en retirant la tige 21 au fur et à mesure de la fixation des portions de l'implant 1 aux tissus, afin de permettre le passage de la partie 5a au travers de la fente 20.

Ainsi que le montre la figure 12, l'implant 2 peut présenter également, en regard de chaque fil de suture 30, sur chacun des côtés de la pièce 10 situés de part et d'autre de la partie 5b, deux bobinots 22 montés sur lui. Chaque brin d'un fil de suture 30 est destiné à être enroulé sur le bobinot 22 situé sur le même côté que lui et l'aiguille de suture est destinée à être maintenue sur ce bobinot 22 le temps qu'elle soit utilisée. Chaque bobinot 22 peut avoir une forme circulaire ou avoir une forme ovale adaptée à la réception de l'aiguille 31.

L'invention fournit ainsi un dispositif d'annuloplastie utilisable par voie mini-invasive, notamment pour la reconstruction de valves cardiaques, qui permet une annuloplastie par voie très peu invasive, c'est-à-dire par une ou plusieurs voies d'abord formées par des passages ayant de l'ordre de un à deux centimètres de diamètre.

Il va de soi que l'invention n'est pas limitée à la forme de réalisation décrite ci-dessus à titre d'exemple mais qu'elle en embrasse, au contraire, toutes les variantes de réalisation. Ainsi, l'implant pourrait avoir une structure élastique et présenter la forme courbe précitée à l'état non déformé de cette structure, ou être réalisé en alliage à mémoire de forme tel qu'un alliage Nickel-titane connu sous la dénomination "NITINOL".

## Revendications

1. Dispositif d'annuloplastie utilisable par voie mini-invasive, notamment pour la reconstruction de valves cardiaques, comprenant un implant conformé pour pouvoir être suturé à des tissus afin de réaliser l'annuloplastie ; **caractérisé**
- **en ce que** l'implant (1) a une structure allongée et déformable, telle qu'il peut prendre d'une part une forme allongée pour son introduction dans le corps du patient par un passage de diamètre réduit, de l'ordre d'un à deux centimètres, et d'autre part une forme courbe adaptée à la réalisation de l'annuloplastie, et
- **en ce que** le dispositif comprend un instrument tubulaire (2) propre à recevoir au moins partiellement ledit implant (1) en lui, qui présente une rigidité telle qu'il permet l'introduction de l'implant (1) dans le corps du patient par ledit passage ; cet instrument (2) comprend une ouverture (12, 13) au niveau de sa partie distale, permettant l'accès à l'implant (1), et comprend des moyens (15) de calage en rotation de l'implant (1) par rapport à lui, des moyens (15) de maintien de l'implant (1) par rapport à lui, et des moyens de repérage de son orientation angulaire à l'intérieur du corps du patient.

2. Dispositif selon la revendication 1, **caractérisé en ce que** l'implant (1) comprend un corps (5) ayant une structure souple non élastique, et au moins un cordon (6) relié audit corps (5), au voisinage de l'une des extrémités de ce dernier ; chaque cordon (6) s'étend sur un côté longitudinal de ce corps (5) jusqu'à un emplacement éloigné de ladite extrémité, est monté coulissant par rapport à ce corps (5) et par rapport audit emplacement, et présente une longueur telle qu'une traction peut être opérée sur lui après la suture dudit corps (5) auxdits tissus ; l'ensemble est conformé de telle sorte qu'une traction peut être opérée sur chaque cordon (6) afin de réduire la longueur dudit corps (5), en fronçant ladite structure, de manière à réduire la circonférence de l'implant (1 ) et, ce faisant, à réaliser l'annuloplastie.

3. Dispositif selon la revendication 2, **caractérisé en ce que** ledit corps (5) est constitué par une tresse de matière textile, et **en ce que** chaque cordon (6) passe à l'intérieur de cette tresse.

4. Dispositif selon la revendication 2 ou la revendication 3, **caractérisé en ce que** l'implant (1) comprend deux cordons (6), dont un est relié à une extrémité du corps (5) et l'autre à l'autre extrémité de ce corps (5), et **en ce que** les deux cordons (6) s'étendent chacun sur sensiblement la moitié du corps (5), jusqu'à des emplacements situés à proximité l'un de l'autre sensiblement dans la zone médiane de ce corps (5).

5. Dispositif selon l'une des revendications 2 à 4, **caractérisé en ce que** l'instrument (2) présente une encoche latérale (12) au voisinage de son extrémité distale, qui communique par une fente (14) avec une ouverture distale (13) de cet instrument (2), la profondeur de cette encoche (12) étant telle qu'elle permet de découvrir le côté longitudinal du corps (5) destiné, après suture, à être situé radialement vers l'extérieur de l'annuloplastie à réaliser, mais telle qu'elle recouvre le côté longitudinal du corps (5) destiné à être situé sur le côté radialement intérieur de cette annuloplastie, c'est-à-dire sur le côté sur lequel se trouvent le ou lesdits cordons (6).

6. Dispositif selon la revendication 5, **caractérisé en ce que** des moyens de maintien tels que précités sont situés de part et d'autre de l'encoche (12).

7. Dispositif selon la revendication 5 ou la revendication 6, **caractérisé en ce que** l'instrument (2) comprend deux pièces tubulaires (10, 11) dont une première (10) est engagée dans la deuxième (11) ; ladite première pièce tubulaire (10) comporte des dents (15) au niveau de son extrémité distale, mobiles radialement entre une position normale, radialement externe, dans laquelle elles autorisent le coulissement de l'implant (1) et une position radialement interne, dans laquelle elles serrent l'implant (1) entre elles et empêchent ce coulissement, ces dents (15) étant conformées de telle sorte que leurs faces radialement extérieures fassent saillie, dans ladite position normale, au-delà de la face extérieure de ladite première pièce tubulaire (10) ; la deuxième pièce tubulaire (11) peut coulisser axialement par rapport à la première pièce tubulaire (10), entre une position effacée, dans laquelle elle ne porte pas contre lesdites faces radialement extérieures des dents, et une position active, dans laquelle elle porte contre ces faces radialement extérieures et déplace les dents dans leur position radialement interne.

8. Dispositif selon l'une des revendications 1 à 4, **caractérisé en ce que** le corps (5) de l'implant (1) présente, vu en coupe transversale, une partie tubulaire (5a) et une partie plane (5b) s'étendant radialement par rapport à cette partie tubulaire (5a) ; l'instrument (2) comprend une pièce tubulaire (10) qui présente une fente latérale (20) aménagée dans sa partie distale, et comprend une tige (21) pouvant être engagée dans cette pièce tubulaire (10) ; ladite partie tubulaire (5a) du corps de l'implant est engagée à l'intérieur de cette pièce tubulaire (10) de l'instrument (2) et reçoit ladite tige (21) en elle tandis que ladite partie plane (5b) traverse ladite fente (20) et débouche sur l'extérieur de ladite pièce tubulaire de l'instrument (2).

9. Dispositif selon la revendication 8, **caractérisé en ce que** l'instrument (2) comprend deux pièces tubulaires, à savoir la pièce tubulaire (10) précitée et une pièce tubulaire externe, dans laquelle sont engagés la pièce tubulaire (10) précitée et l'implant (1) placé dans celle-ci.

10. Dispositif selon la revendication 8 ou la revendication 9, **caractérisé en ce que** la portion de ladite partie plane (5b) du corps de l'implant s'étendant au-delà de l'instrument (2) comprend les moyens de fixation (30) de l'implant aux tissus, qui sont pré-positionnés sur elle.

11. Dispositif selon la revendication 10, **caractérisé en ce que** les moyens de fixation sont constitués par des fils de suture (30) et **en ce que** l'implant (1) présente, en regard de chaque fil de suture (30), au moins un bobinot (22) monté sur lui, sur lequel le fil de suture (30) est enroulé.

12. Dispositif selon la revendication 11, **caractérisé en ce que** les fils de suture (30) traversent ladite partie plane (5b), et **en ce que** l'instrument présente un bobinot (22) sur chacun de ses côtés situés de part et d'autre de cette partie plane (5b), chacun des bobinots (22) recevant l'un des deux brins du fil de suture (30).

13. Dispositif selon la revendication 1, **caractérisé en ce que** l'implant a une structure élastique et présente la forme courbe précitée à l'état non déformé de cette structure, cet implant étant déformé élastiquement lors de son engagement dans l'instrument puis retrouve sa forme courbe au fur et à mesure de sa délivrance par l'instrument.

14. Dispositif selon la revendication 1, **caractérisé en ce que** l'implant est réalisé en alliage à mémoire de forme tel qu'un alliage Nickel-titane connu sous la dénomination "NITINOL".

## Claims

1. Annuloplasty device usable by a minimally invasive route, in particular for heart valve reconstruction, comprising an implant shaped to be suturable to tissues in order to create the annuloplasty ; **characterized in that**
- the implant (1) has an elongate, deformable structure so that it can assume an elongate shape for insertion into the body of the patient through a small-diameter passage, approximately 1 to 2 cm in diameter, and a curved shape adapted for creating the annuloplasty, and
- the device has a tubular instrument (2) able to receive said implant (1) at least partially within itself, which is sufficiently rigid to allow insertion of implant (1) into the body of the patient through said passage ; this instrument (2) has an opening (12, 13) at its distal part enabling access to implant (1) and comprises means (15) for rotationally locking the implant (1) relative thereto, means (15) for holding implant (1) relative thereto, and means for detecting its angular orientation inside the body of the patient.

2. Device according to Claim 1, **characterized in that** implant (1) has a body (5) with a non-elastic flexible structure, and at least one cord (6) connected to said body (5) in the vicinity of one end of the latter ; each cord (6) extends over one lengthwise side of this body (5) up to a location remote from said end, is slidably mounted relative to this body (5) and relative to said location, and has a length such that traction may be exerted thereon once body (5) has been sutured to said tissues ; the whole is shaped such that traction can be exerted on each cord (6) to reduce the length of said body (5) by puckering said structure so as to reduce the circumference of implant (1) and hence create the annuloplasty

3. Device according to Claim 2, **characterized in that** said body (5) is comprised of a braid of textile material and **in that** each cord (6) passes inside this braid.

4. Device according to Claim 2 or Claim 3, **characterized in that** the implant (1) has two cords (6) one of which is connected to one end of body (5) and the other to the other end of this body (5) and **in that** the two cords (6) each extend over substantially half of body (5) up to locations near each other substantially in the median area of this body (5).

5. Device according to one of Claims 2 to 4, **characterized in that** the instrument (2) has a lateral notch (12) in the vicinity of its distal end that communicates via a slot (14) with a distal opening (13) in this instrument (2), the depth of said notch (12) being such as to uncover the lengthwise side of body (5) that is destined, after the suturing, to be located radially outside the annuloplasty to be created, but such that it covers the lengthwise side of body (5) destined to be located on the radially inner side of this annuloplasty, namely on the side on which the cord or cords (6) are located.

6. Device according to Claim 5, **characterized in that** the holding means referred to above are located on either side of notch (21).

7. Device according to Claim 5 or Claim 6, **characterized in that** the instrument (2) has two tubular parts (10, 11) of which the first (10) is engaged in the second (11) ; said first tubular part (10) has teeth (15) at its distal end that are movable radially between a normal radially outer position in which they allow implant (1) to slide and a radially inner position in which they grip the implant (1) between them and prevent this sliding, said teeth (15) being shaped such that their radially outer faces project, in said normal position, beyond the outer face of said first tubular part (10) ; the second tubular part (11) can slide axially relative to the first tubular part (10) between a retracted position in which it does not abut said radially outer faces of the teeth, and an active position in which it abuts these radially outer faces, and moves the teeth into their radially inner position.

8. Device according to one of Claims 1 to 4, **characterized in that** the body (5) of implant (1) has, viewed transversally, a tubular part (5a) and a flat part (5b) extending radially relative to said tubular part (5a) ; the instrument (2) has a tubular part (10) that has a lateral slot (20) provided in its distal part, and has a rod (21) that can be engaged in this tubular part (10) ; said tubular part (5a) of the body of the implant is engaged inside said tubular part (10) of instrument (2) and receives said rod (21) therein while said flat part (5b) passes through said slot (20) and extends outside said tubular part of the instrument (2).

9. Device according to Claim 8, **characterized in that** the instrument (2) has two tubular parts, namely the tubular part (10) referred to above and an outer tubular part in which the aforesaid tubular part (10) and the implant (1) placed therein are engaged.

10. Device according to Claim 8 or Claim 9, **characterized in that** the portion of said flat part (5b) of the implant body extending beyond instrument (2) has means (30) for attaching the implant to the tissues, which are pre-positioned on said part.

11. Device according to Claim 10, **characterized in that** the attaching means are comprised of suture threads (30) and **in that** the implant (1) has, opposite each suture thread (30), at least one reel (22) mounted thereon, onto which suture thread (30) is wound.

12. Device according to Claim 1, **characterized in that** the suture threads (30) pass through said flat part (5b) and **in that** the instrument has a reel (22) on each of its sides located on either side of said flat part (5b), each of reels (22) receiving one of the two strands of suture thread (30).

13. Device according to Claim 1, **characterized in that** the implant has an elastic structure and has the aforementioned curved shape in the non-deformed state of this structure, this implant being deformed elastically when it is engaged in the instrument, then returns to its curved shape as it is dispensed by the instrument.

14. Device according to Claim 1, **characterized in that** the implant is made of a shape-memory alloy such as a nickel-titanium alloy known as Nitinol.

## Patentansprüche

1. Annuloplastievorrichtung zur minimal-invasiven Verwendung, insbesondere für die Rekonstruktion von Herzklappen, mit einem zum Aufnähen auf Gewebe ausgebildeten Implantat, um so die Annuloplastie durchzuführen,
**dadurch gekennzeichnet, dass**
- das Implantat (1) eine längliche und verformbare Struktur aufweist, so dass dieses einerseits eine längliche Form zum Einführen in den Körper des Patienten durch einen Durchgang mit einem kleinen Durchmesser in der Größenordnung von einem bis zwei Zentimetern und andererseits eine gekrümmte Form für die Durchführung der Annuloplastie einnehmen kann, und
- dass die Vorrichtung ein zum wenigstens teilweisen Aufnehmen des besagten Implantats (1) geeignetes rohrförmiges Instrument (2) aufweist, welches eine derartige Steifheit hat, dass dieses die Einführung des Implantats (1) in den Körper des Patienten durch den besagten Durchgang erlaubt; dieses Instrument (2) weist in seinem distalen Bereich eine den Zugang zu dem Implantat (1) erlaubende Öffnung (12, 13) und Mittel (15) zur Einstellung der Drehung des Implantats (1) relativ zu diesem, Mittel (15) zum Halten des Implantats (1) relativ zu diesem und Mittel zum Erkennen seiner Winkelstellung im Innern des Körpers des Patienten auf.

2. Vorrichtung nach Anspruch 1, **dadurch gekennzeichnet, dass** das Implantat (1) einen Körper (5) mit einer biegsamen, nicht-elastischen Struktur und wenigstens eine mit dem besagten Körper (5) in der Nähe eines von dessen Enden verbundene Schnur (6) aufweist; jede Schnur (6) erstreckt sich über eine Längsseite dieses Körpers (5) bis zu einer von diesem Ende entfernten Stelle, ist verschiebbar bezüglich dieses Körpers (5) und bezüglich der besagten Stelle angebracht und weist eine derartige Länge auf, dass nach Aufnähen des besagten Körpers (5) auf das besagte Gewebe ein Zug auf diese ausgeübt werden kann; die Anordnung ist derart ausgebildet, dass ein Zug auf jede Schnur (6) ausgeübt werden kann, um die Länge des besagten Körpers (5) durch Falten der besagten Struktur zu reduzieren, so dass der Umfang des Implantats (1) reduziert wird und dadurch die Annuloplastie durchgeführt wird.

3. Vorrichtung nach Anspruch 2, **dadurch gekennzeichnet, dass** der besagte Körper (5) aus einem Geflecht von Textilmaterial besteht und dass jede Schnur (6) sich im Innern dieses Geflechts erstreckt.

4. Vorrichtung nach Anspruch 2 oder 3, **dadurch gekennzeichnet, dass** das Implantat (1) zwei Schnüre (6) aufweist, von denen die eine mit dem einen Ende des Körpers (5) und die andere mit dem anderen Ende dieses Körpers (5) verbunden ist, und dass sich die beiden Schnüre (6) jeweils über im wesentlichen die Hälfte des Körpers (5) bis zu Stellen erstrecken, die sich nahe zueinander im wesentlichen im mittleren Bereich dieses Körpers (5) befinden.

5. Vorrichtung nach einem der Ansprüche 2 bis 4, **dadurch gekennzeichnet, dass** das Instrument (2) eine seitliche Aussparung (12) in der Nähe seines distalen Endes aufweist, die über einen Schlitz (14) mit einer distalen Öffnung (13) dieses Instruments (2) verbunden ist, wobei die Tiefe dieser Aussparung (12) derart ist, dass sie die Freilegung der Längsseite des Körpers (5) erlaubt, die nach dem Aufnähen radial nach außen an der durchzuführenden Annuloplastie sitzen soll, aber auch derart, dass sie die Längsseite des Körpers (5) abdeckt, die sich auf der radial inneren Seite dieser Annuloplastie befinden soll, das heißt auf der Seite, auf der sich die Schnur (6) oder die Schnüre (6) befinden.

6. Vorrichtung nach Anspruch 5, **dadurch gekennzeichnet, dass** die oben genannten Mittel zum Halten sich beiderseits der Aussparung (12) befinden.

7. Vorrichtung nach Anspruch 5 oder 6, **dadurch gekennzeichnet, dass** das Instrument (2) zwei rohrförmige Teile (10, 11) aufweist, von denen das erste Teil (10) im zweiten Teil (11) aufgenommen ist; das besagte erste rohrförmige Teil (10) weist im Bereich seines distalen Endes Zähne (15) auf, die zwischen einer ersten normalen, radial äußeren Stellung, in der sie das Verschieben des Implantats (1) erlauben, und einer radial inneren Stellung, in der sie das Implantat (1) zwischen sich festsetzen und dieses Verschieben verhindern, radial beweglich sind, wobei die Zähne (15) derart ausgebildet sind, dass ihre radial äußeren Flächen in der besagten normalen Stellung von der Außenfläche des besagten ersten rohrförmigen Teils (10) vorstehen; das zweite rohrförmige Teil (11) kann bezüglich des ersten rohrförmigen Teils (10) zwischen einer Nullstellung, in der es nicht an den besagten radial äußeren Flächen der Zähne anliegt, und einer aktiven Stellung, in der es gegen diese radial äußeren Flächen anliegt und die Zähne in ihre radial innere Stellung verschiebt, axial gleiten.

8. Vorrichtung nach einem der Ansprüche 1 bis 4, **dadurch gekennzeichnet, dass** der Körper (5) des Implantats (1), im Querschnitt gesehen, einen rohrförmigen Teil (5a) und einen ebenen Teil (5b) aufweist, der sich radial bezüglich dieses rohrförmigen Teils (5a) erstreckt; das Instrument (2) weist ein rohrförmiges Teil (10), das mit einem in seinem distalen Teil angebrachten seitlichen Schlitz (20) versehen ist, und einen Stift (21) auf, der in diesem rohrförmigen Teil (10) aufgenommen werden kann; der besagte rohrförmige Teil (5a) des Körpers des Implantats (1) ist im Innern dieses rohrförmigen Teils (10) des Instruments (2) aufgenommen und nimmt den besagten Stift (21) in sich auf, während der ebene Teil (5b) den besagten Schlitz (20) durchquert und außen auf dem besagten rohrförmigen Teil des Instruments (2) mündet.

9. Vorrichtung nach Anspruch 8, **dadurch gekennzeichnet, dass** das Instrument (2) zwei rohrförmige Teile aufweist, nämlich das vorgenannte rohrförmige Teil (10) und ein äußeres rohrförmiges Teil, in dem das vorgenannte rohrförmige Teil (10) und das darin befindliche Implantat (1) aufgenommen sind.

10. Vorrichtung nach Anspruch 8 oder 9, **dadurch gekennzeichnet, dass** der sich über das Instrument (2) hinausstehende Teil des besagten ebenen Teils (5b) des Körpers des Implantats (1) die darauf vorpositionierten Mittel zur Befestigung (30) des Implantats (1) am Gewebe aufweist.

11. Vorrichtung nach Anspruch 10, **dadurch gekennzeichnet, dass** die Mittel zur Befestigung aus Nähfäden (30) bestehen und dass das Implantat (1) für jeden Nähfaden (30) eine darauf angebrachte Spule (22) aufweist, auf der der Nähfaden (30) aufgerollt ist.

12. Vorrichtung nach Anspruch 11, **dadurch gekennzeichnet, dass** die Nähfäden (30) den besagten ebenen Teil (5b) durchqueren und dass das Instrument (2) auf jeder seiner Seiten beiderseits dieses ebenen Teils (5b) eine Spule (22) aufweist, wobei jede Spule (22) den einen der beiden Stränge des Nähfadens (30) aufnimmt.

13. Vorrichtung nach Anspruch 1, **dadurch gekennzeichnet, dass** das Implantat (1) eine elastische Struktur hat und die vorgenannte gekrümmte Form im nicht verformbaren Zustand dieser Struktur aufweist, wobei dieses Implantat (1) bei seiner Aufnahme in das Instrument (2) elastisch verformt wird und dann seine gekrümmte Form im Maß seiner Zuführung von dem Instrument (2) wieder einnimmt.

14. Vorrichtung nach Anspruch 1, **dadurch gekennzeichnet, dass** das Implantat (1) als Memory-Legierung ausgebildet ist, wie beispielsweise als Nickel-Titan-Legierung unter der Bezeichnung "NITINOL".
